# EUROPEAN PATENT APPLICATION

(11) **EP 4 035 726 A1**
(43) Date of publication of application: **03.08.2022**
(21) Application number: 19946283.9
(22) Date of filing: 26.12.2019
(51) Int. Cl.: A61N 1/36, A61B 5/313

(54) **ELECTRICAL STIMULATION THERAPEUTIC DEVICE AND ELECTRICAL STIMULATION THERAPEUTIC METHOD**

(30) Priority: 24.09.2019 JP 2019173397
(71) Applicant: Otsuka Techno Corporation, Naruto-shi, Tokushima 771-0360 (JP); Ito Co., Ltd., Tokyo 113-0001 (JP)
(72) Inventor: HISAMOTO, Takashi, Naruto-shi, Tokushima 771-0360 (JP); MUKAI, Yoichi, Naruto-shi, Tokushima 771-0360 (JP); MASUDA, Tetsuya, Naruto-shi, Tokushima 771-0360 (JP); KAKUYAMA, Hiroki, Naruto-shi, Tokushima 771-0360 (JP); MAEDA, Koji, Tokyo 113-0001 (JP); UEDA, Shuhei, Tokyo 113-0001 (JP)
(74) Representative: Hoffmann Eitle
(86) International application number: PCT/JP2019/051118
(87) International publication number: WO 2021/059545

(57) **Abstract**

An electrical stimulation therapeutic device includes a pair of application electrodes which are disposed at the back of a sacral bone of a person to be treated and supplies an electrical stimulation signal from the back of the sacral bone, a detection electrode which is disposed on a surface of a toe of the person to be treated and detects a myoelectric signal of the toe, a display portion which determines whether the myoelectric signal of the toe is generated in response to the stimulation signal, and a myoelectric signal processing portion which processes the myoelectric signal detected by the detection electrode to display it visually on the display portion, in which the myoelectric signal processing portion does not detect the myoelectric signal of the toe during a predetermined detection stop period of time from output of the stimulation signal, and the detection stop period of time is set on the basis of a quotient (x/v) obtained when a distance (x) from a sacral bone of a human body to a surface of a toe is divided by a transmission velocity (v) of a nerve which passes through the sacral bone of the human body or the vicinity of the sacral bone.

## Description

### Technical Field

The present invention relates to a device that is used for electrical stimulation therapy and an electrical stimulation therapeutic method that uses this device.

### Background Art

As an example of a device used for electrical stimulation therapy, there has been so far proposed a device for treating a urination disorder.

For example, Patent Literature 1 has disclosed a urination disorder treatment device which includes a pair of application electrodes and a detection electrode. In the urination disorder treatment device, a stimulation pulse from the pair of application electrodes is compared with a detection pulse of a toe to determine whether a nerve which passes through a sacral bone or the vicinity of the sacral bone is properly stimulated by the stimulation pulse. The detection pulse is generated by responses of a tibial nerve and/or a peroneal nerve which are connected to the nerve which passes through the sacral bone or the vicinity of the sacral bone by way of an ischiadic nerve and extend up to the tip of a toe.

Further, for example, Patent Literature 2 has disclosed a myogenic potential monitoring device including a surface electrode which detects a myogenic potential, an amplifier which amplifies a myogenic potential signal detected by the surface electrode, a band pass filter which extracts a myogenic potential component to be monitored by an amplified waveform, a rectifier which rectifies the component that passed through the band pass filter, a level setting device which is provided on the rectifier, an integrator by which rectified signals are integrated for a certain sampling time, an AD converter which converts the signal that has been integrated to a digital signal, a counter which counts the converted digital signals, a latch driver which holds the counted digital signals and drives a display, and a controller which controls an operation timing of the integrator, the AD converter, the counter, the latch driver and the display.

### Citation List

### Patent Literatures

Patent Literature 1: Japanese Patent No. 6488498
Patent Literature 2: Japanese Unexamined Utility Model Application Publication No. S58-10704

### Summary of Invention

### Technical Problem

According to the urination disorder treatment device of Patent Literature 1, on the basis of myoelectric data of a toe, it is possible to confirm whether the stimulation signal is appropriately transmitted from the application electrodes to the nerve which passes through the sacral bone or the vicinity of the sacral bone.

However, there is a case that, when the electrical stimulation is given from the application electrodes to the back of the sacral bone of a human body, a stimulation voltage is transmitted to a toe as noise to cause in a superimposed manner a large noise in the myoelectric data of the toe. Therefore, it is desired to be able to detect the myoelectric data of a toe more accurately.

For example, an evaluation is made for a method in which, during a period of time when an electrical stimulation is given from an application electrode to a human body, detection of myoelectric data of a toe is stopped to eliminate noise resulting from a stimulation voltage. However, this method is insufficient. This is because, although the noise is less likely to be detected by the stop of detecting the myoelectric data during the period of time, in general, a digital filter and an analog filter, each of which has a time constant, are provided on a signal processing circuit of myoelectric data (refer to Patent Literature 2, for example). Therefore, there is a case that, even if detection of the myoelectric data is simply stopped only during a pulse width of the electrical stimulation, noise may be detected after an elapsed time of the stop due to the influence of the time constant.

Thus, an object of the present invention is to provide an electrical stimulation therapeutic device capable of accurately confirming whether an application electrode to be disposed at the back of a sacral bone is appropriately attached and an electrical stimulation therapeutic method which uses the device.

### Solution to Problem

An electrical stimulation therapeutic device according to one aspect of the present invention includes a pair of application electrodes which are disposed at the back of a sacral bone of a person to be treated and supplies an electrical stimulation signal from the back of the sacral bone, a detection electrode which is disposed on a surface of a toe of the person to be treated and detects a myoelectric signal of the toe, a display portion which determines whether the myoelectric signal of the toe is generated in response to the stimulation signal, and a myoelectric signal processing portion which processes the myoelectric signal detected by the detection electrode and displays it visually on the display portion and in which the myoelectric signal processing portion does not detect the myoelectric signal of the toe during a predetermined detection stop period of time from output of the stimulation signal and the detection stop period of time is set on the basis of a quotient (x/v) obtained when a distance (x) from a sacral bone of a human body to a surface of a toe is divided by a transmission velocity (v) of a nerve which passes through the sacral bone of the human body or the vicinity of the sacral bone.

### Effects of Invention

In an electrical stimulation therapeutic device according to one aspect of the present invention, during a detection stop period of time which is set on the basis of a quotient (x/v) obtained when a distance (x) from a sacral bone of a human body to a surface of a toe is divided by a transmission velocity (v) of a nerve passing through the sacral bone of the human body or the vicinity of the sacral bone, a myoelectric signal of the toe is not detected by a myoelectric signal processing portion.

During the detection stop period of time, a stimulation signal given to the human body does not reach a toe by way of the nerve. That is, the myoelectric signal which is detected during a time before the stimulation signal reaches the toe by way of the nerve after application of an electrical stimulation is not a signal which carries information necessary for myoelectric monitoring but noise. Therefore, the myoelectric signal of the toe is not detected by the myoelectric signal processing portion during the detection stop period of time, thus making it possible to eliminate the noise of the myoelectric signal. Thereby, it is possible to accurately detect the myoelectric signal which reaches the toe via the nerve passing through a sacral bone or the vicinity of the sacral bone and is generated in response to the signal which carries information necessary for myoelectric monitoring. Therefore, in the electrical stimulation therapeutic device according to one aspect of the present invention, it is possible to accurately confirm whether the stimulation signal is appropriately transmitted from the application electrodes to the nerve which passes through the sacral bone or the vicinity of the sacral bone on the basis of myoelectric data of the toe.

### Brief Description of Drawings

[FIG. 1] FIG. 1 is a side cross-sectional view of a human body for describing innervation of urination.
[FIG. 2] FIG. 2 is a rear view of the human body for describing innervation of urination.
[FIG. 3A] FIG. 3A is a drawing for describing a mechanism of urination.
[FIG. 3B] FIG. 3B is a drawing for describing a mechanism of urination.
[FIG. 4] FIG. 4 is a schematic view which shows an electrical stimulation therapeutic device according to a preferred embodiment of the present invention.
[FIG. 5] FIG. 5 is a front view of an electrode pad of the electrical stimulation therapeutic device.
[FIG. 6] FIG. 6 is a rear view of the electrode pad of the electrical stimulation therapeutic device.
[FIG. 7] FIG. 7 is a cross-sectional view of the electrode pad of the electrical stimulation therapeutic device and shows a cross section taken along line VII-VII in FIG. 5.
[FIG. 8] FIG. 8 is a drawing which shows a state that the electrode pad is attached.
[FIG. 9] FIG. 9 is a drawing which shows a disposition of a detection electrode on monitoring myoelectric data.
[FIG. 10] FIG. 10 is a block diagram which shows an electric configuration of the electrical stimulation therapeutic device.
[FIG. 11] FIG. 11 is a flowchart which shows monitoring of myoelectric data of the electrical stimulation therapeutic device.
[FIG. 12] FIG. 12 is a drawing for describing one example of a stimulation pulse and one example of a pattern of a myoelectric signal.
[FIG. 13] FIG. 13 is a drawing which shows a pattern of the myoelectric signal before masking processing.
[FIG. 14] FIG. 14 is a drawing which shows a pattern (first example) of the myoelectric signal after masking processing.
[FIG. 15] FIG. 15 is a drawing which shows a pattern (second example) of the myoelectric signal after masking processing.
[FIG. 16] FIG. 16 is a drawing for describing an integration of the myoelectric signal by an integrator.
[FIG. 17] FIG. 17 is a drawing for describing elimination of a change in reference line of an electromyogram.
[FIG. 18] FIG. 18 is a drawing for describing a level indication of the electromyogram.
[FIG. 19] FIG. 19 is a schematic view of an electrical stimulation therapeutic device according to another preferred embodiment of the present invention. Description of Embodiments

### <Preferred Embodiments of the Present Invention>

First, a description will be given by listing the preferred embodiments of the present invention.

An electrical stimulation therapeutic device according to a preferred embodiment of the present invention includes a pair of application electrodes which are disposed at the back of a sacral bone of a person to be treated and supplies an electrical stimulation signal from the back of the sacral bone, a detection electrode which is disposed on a surface of a toe of the person to be treated and detects a myoelectric signal of the toe, a display portion which determines whether the myoelectric signal of the toe is generated in response to the stimulation signal, and a myoelectric signal processing portion which processes the myoelectric signal detected by the detection electrode and visually displays it on the display portion, in which the myoelectric signal processing portion does not detect the myoelectric signal of the toe for a predetermined detection stop period of time from output of the stimulation signal, and the detection stop period of time is set on the basis of a quotient (x/v) obtained when a distance (x) from a sacral bone of a human body to a surface of a toe is divided by a transmission velocity (v) of a nerve which passes through the sacral bone of the human body or the vicinity of the sacral bone.

According to this configuration, during the detection stop period of time set on the basis of the quotient (x/v) obtained when the distance (x) from the sacral bone of the human body to the surface of the toe is divided by the transmission velocity (v) of the nerve which passes through the sacral bone of the human body or the vicinity of the sacral bone, the myoelectric signal of the toe is not detected by the myoelectric signal processing portion.

During the detection stop period of time, the stimulation signal given to the human body does not reach the toe via the nerve. That is, the myoelectric signal which is detected after application of the electrical stimulation up to a time when the stimulation signal thereof reaches the toe via the nerve is not a signal which carries information necessary for myoelectric monitoring but noise. Therefore, during the detection stop period of time, the myoelectric signal of the toe is not detected by the myoelectric signal processing portion, thus making it possible to eliminate the noise of the myoelectric signal. Thereby, it is possible to accurately detect the myoelectric signal which reaches the toe via the nerve that passes through the sacral bone or the vicinity of the sacral bone and which is generated in response to the signal that carries information necessary for myoelectric monitoring. Therefore, in the electrical stimulation therapeutic device according to one aspect of the present invention, it is possible to accurately confirm on the basis of myoelectric data of the toe whether the stimulation signal is appropriately transmitted from the application electrodes to the nerve which passes through the sacral bone or the vicinity of the sacral bone,

An electrical stimulation therapeutic device according to a preferred embodiment of the present invention may include a switch portion which turns on or off a connection of the detection electrode with the myoelectric signal processing portion, and a myoelectric signal selecting portion which controls the switch portion so that the connection of the detection electrode with the myoelectric signal processing portion can be turned off during the detection stop period of time and also the connection of the detection electrode with the myoelectric signal processing portion can be turned on at the end of the detection stop period of time.

According to this configuration, at the end of the detection stop period of time, the connection of the detection electrode with the myoelectric signal processing portion is turned on, thus making it possible to stabilize a potential of the myoelectric signal at the end of the detection stop period of time.

In an electrical stimulation therapeutic device according to a preferred embodiment of the present invention, the myoelectric signal selecting portion may make a potential of the switch portion into a reference potential or a pre-held drift potential or may make the switch portion into an open state during the detection stop period of time.

An electrical stimulation therapeutic device according to a preferred embodiment of the present invention may include an integrator by which, before processing of the myoelectric signal in the myoelectric signal processing portion, myoelectric signals detected by the detection electrode are integrated for a predetermined sampling time and the integrated signal is output in the myoelectric signal processing portion as a first signal.

According to this configuration, an amplitude, a peak and the like of the myoelectric signal (first signal) can be easily detected, thus making it possible to efficiently process the myoelectric signal in the myoelectric signal processing portion.

In an electrical stimulation therapeutic device according to a preferred embodiment of the present invention, the myoelectric signal processing portion may determine an amplitude of the first signal on the basis of a difference between an averaged value of a first section of the first signal for a predetermined first period of time and an averaged value of a second section of the first signal for a predetermined second period of time before and after the first section.

According to this configuration, it is possible to eliminate, for example, a change arising from movements, etc., of a human body, a change due to introduction of a commercial alternating current and a change in reference line (drift) due to noise introduction, etc., from an electric appliance around the human body. As a result, an amplitude, a peak and the like of the myoelectric signal (first signal) can be detected accurately.

In an electrical stimulation therapeutic device according to a preferred embodiment of the present invention, the first section and the second section of the first signal may be averaged by addition arithmetic average processing or moving average processing.

An electrical stimulation therapeutic device according to a preferred embodiment of the present invention includes a stimulation signal output portion which supplies the stimulation signal to the pair of application electrodes and also demarcates an amplitude of the stimulation signal in a stepwise manner to indicate a level of the amplitude of the stimulation signal on the display portion, and the myoelectric signal processing portion may demarcate the first section of the first signal which is equal to or larger than a predetermined amplitude in a stepwise manner and indicate a level of the amplitude of the first section on the display portion.

According to this configuration, a myoelectric signal, an amplitude of which is less than a predetermined magnitude, is not displayed, by which a relationship between a stimulation signal and the myoelectric signal generated in response to the signal can be easily confirmed.

An electrical stimulation therapeutic method according to a preferred embodiment of the present invention is the electrical stimulation therapeutic method which uses an electrical stimulation therapeutic device including a pair of application electrodes which are disposed at the back of a sacral bone of a person to be treated and supplies an electrical stimulation signal from the back of the sacral bone, a detection electrode which is disposed on a surface of a toe of the person to be treated and detects a myoelectric signal of the toe, a display portion which determines whether the myoelectric signal of the toe is generated in response to the stimulation signal and a myoelectric signal processing portion which processes the myoelectric signal detected by the detection electrode and displays it visually on the display portion, in which the myoelectric signal processing portion does not detect the myoelectric signal of the toe during a predetermined detection stop period of time from output of the stimulation signal, and the detection stop period of time is set on the basis of a quotient (x/v) obtained when a distance (x) from a sacral bone of a human body to a surface of a toe is divided by a transmission velocity (v) of a nerve which passes through the sacral bone of the human body or the vicinity of the sacral bone, and which includes a step in which the pair of application electrodes are disposed at the back of the sacral bone of the person to be treated, a step in which the detection electrode is disposed on the surface of the toe of the person to be treated, a step in which the electrical stimulation signal is supplied to the back of the sacral bone from the pair of application electrodes and a step in which, during supply of the stimulation signal from the pair of application electrodes, whether the myoelectric signal of the toe is generated in response to the stimulation signal is determined by visually confirming the display portion.

An electrical stimulation therapeutic method according to a preferred embodiment of the present invention may include a step in which, where the myoelectric signal of the toe is determined not to be generated in response to the stimulation signal by visually confirming the display portion, supply of the stimulation signal from the pair of application electrodes is stopped.

### <Detailed Description of Preferred Embodiments of the Present Invention>

Next, the preferred embodiments of the present invention will be described in detail by referring to the attached drawings.

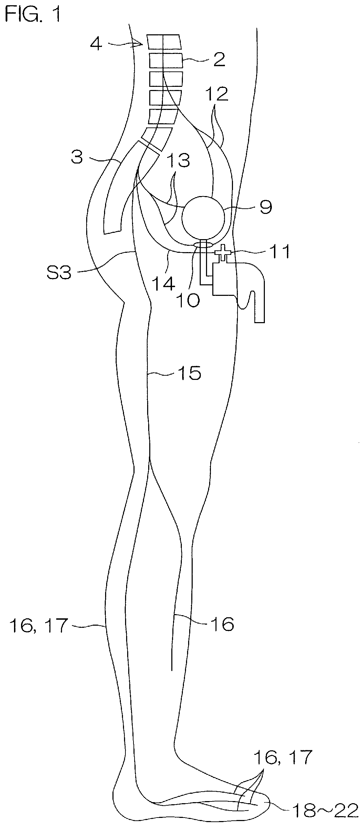

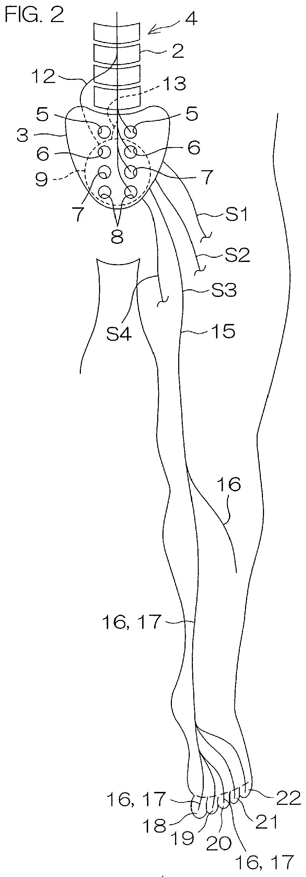

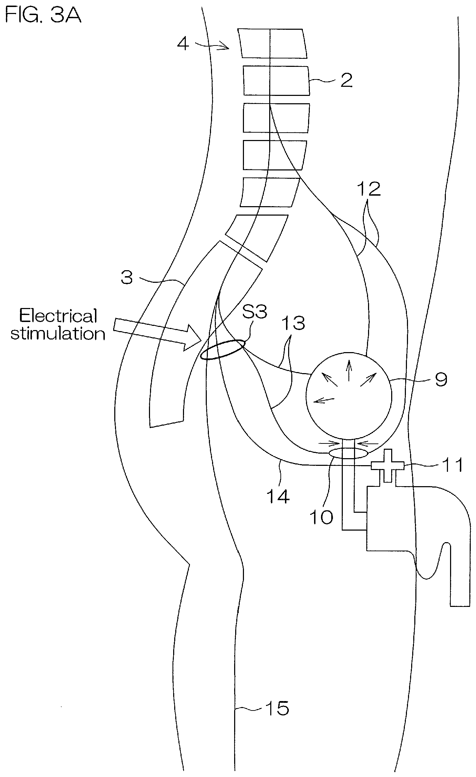

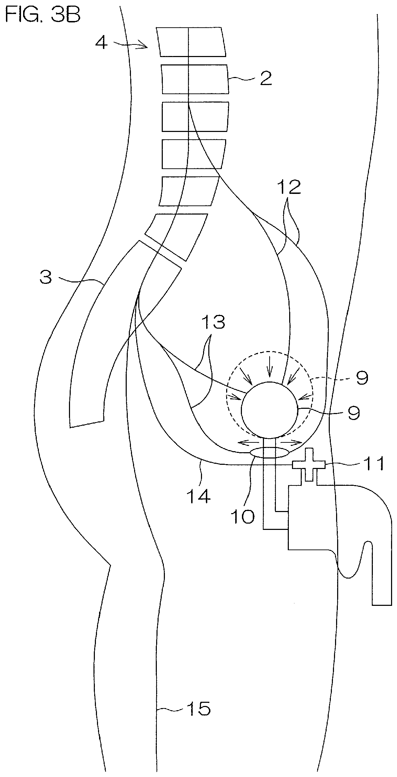

FIG. 1 is a side cross-sectional view of a human body 1 for describing innervation of urination. FIG. 2 is a rear view of the human body 1 for describing innervation of urination. FIG. 3A and FIG. 3B are each a drawing for describing a mechanism of urination. In FIG. 1 to FIG. 3A, B, of various sites of the human body 1, shown are only sites which are necessary for describing treatment by an electrical stimulation therapeutic device 31 according to a preferred embodiment of the present invention, with a description of other sites being omitted here.

The human body 1 has a vertebral column 4 which includes a lumbar vertebra 2, a sacral bone 3 and the like. The sacral bone 3 assumes a substantially inverted triangular shape, normally having four foramina on both sides symmetrically from above a first sacral foramen 5, a second sacral foramen 6, a third sacral foramen 7 and a fourth sacral foramen 8.

Further, the human body 1 has a bladder 9, an internal urethral sphincter 10 and an external urethral sphincter 11 as sites (organs and muscles) involved in collecting and discharging urine. These sites 9 to 11 are neurologically controlled to collect and discharge urine in the human body 1.

In the human body 1, nerves mainly contributing to collection and discharge of urine are a hypogastric nerve (sympathetic nerve) 12, a pelvic nerve (parasympathetic nerve) 13 and a pudic nerve (somatic nerve) 14.

The hypogastric nerve 12 contributes to suppression of urination (urine collection) and is connected to the bladder 9 and the internal urethral sphincter 10. The pelvic nerve 13 contributes to the initiation of urination and is connected to the bladder 9 and the internal urethral sphincter 10. The pudic nerve 14 is connected to the external urethral sphincter 11.

As shown in FIG. 3A, in the human body 1, first, the bladder 9 (detrusor muscle) is relaxed by a signal from the hypogastric nerve 12, by which urine can be easily collected in the bladder 9 and the internal urethral sphincter 10 contracts. Thereby, urine is prevented from being discharged but collected inside the bladder 9. On the other hand, as shown in FIG. 3B, the bladder 9 (detrusor muscle) contracts by a signal from the pelvic nerve 13 and the internal urethral sphincter 10 is also relaxed. Thereby, urine is discharged outside the bladder 9. Then, the external urethral sphincter 11 as a voluntary muscle is relaxed by a command from the brain of the human body 1 (one's own volition) by way of the pudic nerve 14 as a somatic nerve, and an abdominal muscle pressure is applied to discharge urine.

As described above, if the hypogastric nerve 12 and the pelvic nerve 13 are both normally engaged in activity to appropriately contract and relax the bladder 9 and the internal urethral sphincter 10, urine is collected and discharged normally. However, for example, when the hypogastric nerve 12 is activated at a lower level or the pelvic nerve 13 is activated excessively, the bladder 9 is more likely to contract and the internal urethral sphincter 10 is more likely to relax. As a result, it becomes difficult to collect urine in the bladder 9, which may trigger onset of a urination disorder such as urine collection failure (overactive bladder).

Thus, in this preferred embodiment, as shown in FIG. 3A, an electrical stimulation signal is given to the skin on the sacral bone 3 from the back of the sacral bone 3, thereby stimulating the sacral plexus. More specifically, as shown in FIG. 2, a first sacral nerve S1 which passes through the first sacral foramen 5, a second sacral nerve S2 which passes through the second sacral foramen 6, a third sacral nerve S3 which passes through the third sacral foramen 7 and a fourth sacral nerve S4 which passes through the fourth sacral foramen 8 are stimulated. Thereby, for example, as shown in FIG. 3A, the third sacral nerve S3 is stimulated to suppress innervation which causes the bladder 9 to contract by the pelvic nerve 13. Further, this electrical stimulation is also transmitted to the hypogastric nerve 12, thereby accelerating innervation which allows the bladder 9 to be relaxed by the hypogastric nerve 12. As a result, suppression of the pelvic nerve 13 is well-balanced with acceleration of the hypogastric nerve 12, by which the bladder 9 is appropriately relaxed to improve an overactive bladder.

Next, the above-described electrical stimulation is also transmitted to the nerves present at sites other than the buttocks and peripheries thereof at which the sacral plexus is found. For example, as shown in FIG. 2, some of the third sacral nerves S3 partially descend the femur as an ischiadic nerve 15 and finally are divided into a peroneal nerve 16 and a tibial nerve 17. The peroneal nerve 16 and the tibial nerve 17 extend up to the toes of the human body 1 (a first toe 18 (big toe), a second toe 19, a third toe 20, a fourth toe 21 and a fifth toe 22 (little toe)) as terminal portions of the ischiadic nerve 15. That is, the peroneal nerve 16 and the tibial nerve 17 of the toes 18 to 22 are connected by way of the ischiadic nerve 15 and the sacral plexus S3 to the hypogastric nerve 12, the pelvic nerve 13 and the pudic nerve 14.

Next, a description will be given of a configuration and operation thereof of an electrical stimulation therapeutic device 31 according to a preferred embodiment of the present invention.

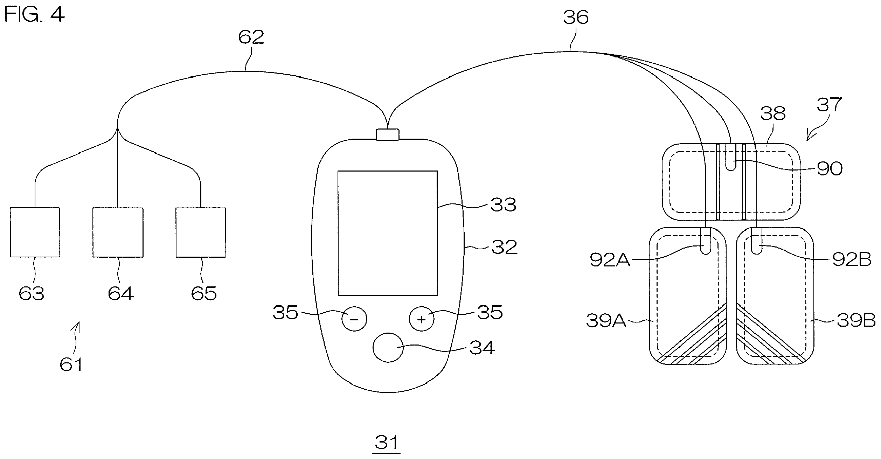

FIG. 4 is a schematic view of an electrical stimulation therapeutic device 31 according to a preferred embodiment of the present invention.

The electrical stimulation therapeutic device 31 is provided, as a physical configuration, with a casing 32 (therapeutic device main-body), a monitor 33 installed on a front surface of the casing 32, a start/stop button 34 installed below the monitor 33, a plurality of operation buttons 35,35, an electrode pad 37 connected to the casing 32 by way of a wiring 36, and a toe electrode 61 which is connected to the casing 32 by way of a toe wiring 62 as an example of the detection electrode of the present invention.

In this preferred embodiment, the casing 32 is formed in a substantially oval-shape and may be, for example, a plastic-made case. Further, although not shown in the drawing, there may be provided, at the back of the casing 32, a removable back cover for housing a battery for a power source of the electrical stimulation therapeutic device 31. The power source of the electrical stimulation therapeutic device 31 may not necessarily be a battery but may be obtained, for example, from an electrical outlet by way of an AC adaptor. The battery may be used together with the outlet.

The monitor 33 may be formed in a rectangular shape so as to be longer along a longitudinal direction of the casing 32 and disposed to be closer to one end of the casing 32 in the longitudinal direction. Further, the monitor 33 may be, for example, a black-and-white or color liquid crystal monitor. The monitor 33 can display, for example, a pulse waveform and a frequency of an electrical stimulation signal by the electrode pad 37, an electrocardiographic waveform and a heart rate of a person to be treated, an error message and the like. Thereby, the person to be treated can easily know the operating state of the electrical stimulation therapeutic device 31. The monitor 33 may also be a touch panel in which a predetermined operating screen is, for example, displayed so that the screen can be operated.

The start/stop button 34 and the plurality of operation buttons 35, 35 may be disposed on the other end side of the casing 32 in the longitudinal direction in relation to the monitor 33.

Further, the operation button 35 may have various functions depending on a type of the electrical stimulation therapeutic device 31. For example, as a memory function of the electrical stimulation therapeutic device 31, a treatment menu which includes a width of a pulse wave (pulse width), a frequency of a stimulation signal and the like suitable for each of a plurality of persons to be treated is stored in the electrical stimulation therapeutic device 31, and the button may be used as a button, etc., operated in reading the treatment menu. It may also be a button which is to be depressed when a person to be treated feels that stimulation is strong in a treatment session which will be described later. The wiring 36 is arranged, for example, with a conducting wire covered with a protective insulation coating.

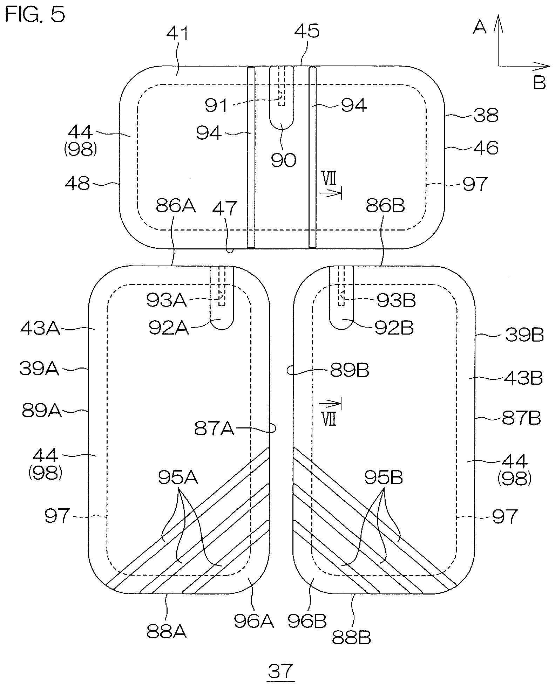

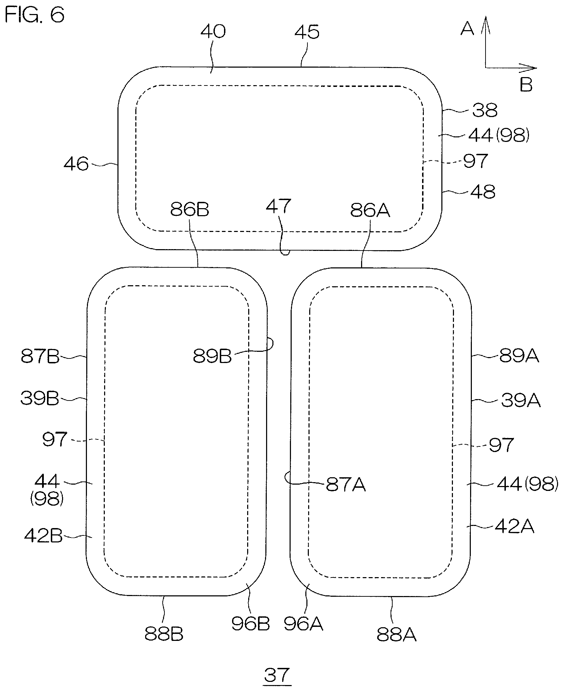

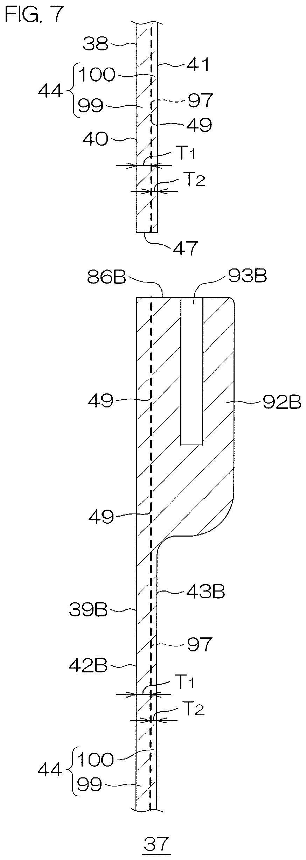

FIG. 5 is a front view of the electrode pad 37 of the electrical stimulation therapeutic device 31. FIG. 6 is a rear view of the electrode pad 37 of the electrical stimulation therapeutic device 31. FIG. 7 is a cross-sectional view of the electrode pad 37 of the electrical stimulation therapeutic device 31, showing a cross section taken along line VII-VII in FIG. 5.

The electrode pad 37 includes an indifferent electrode 38 and a pair of application electrodes 39A, 39B.

The indifferent electrode 38 and the pair of application electrodes 39A, 39B have flexibility by which they can be curved according to bending found when a human body 1 bends (moves). In this preferred embodiment, the indifferent electrode 38 and the pair of application electrodes 39A, 39B are each arranged with a sheet-like (plate-like) rubber base 44 having respectively first surfaces 40, 42A, 42B which face the skin of the human body 1 and second surfaces 41, 43A, 43B at the opposite side of the first surfaces 40, 42A, 42B.

Here, "the sheet-like rubber base 44" means a member which is mainly occupied with a region having a thickness of, for example, 0.5 mm to 2.0 mm. As a matter of course, the rubber base 44 may have a structure, the thickness of which partially exceeds the above range. The above-described structure includes, for example, a first terminal 90 and second terminals 92A, 92B which will be described later.

In this preferred embodiment, the indifferent electrode 38 is formed in a substantially rectangular shape which is horizontally long. The indifferent electrode 38 has a first terminal portion 45, a second terminal portion 46, a third terminal portion 47 and a fourth terminal portion 48, each of which constitutes a side of the rectangular shape.

The first terminal portion 45 is, for example, an upper terminal portion of the indifferent electrode 38 when the indifferent electrode 38 is attached to the human body 1 and opposed to the third terminal portion 47. That is, the third terminal portion 47 is a lower terminal portion of the indifferent electrode 38 when the indifferent electrode 38 is attached to the human body 1. The second terminal portion 46 and the fourth terminal portion 48 couple the first terminal portion 45 and the third terminal portion 47 and are opposed to each other.

The indifferent electrode 38 is, for example, about 9.5 cm in length in a horizontal direction B along the first terminal portion 45 and the third terminal portion 47 and about 5.3 cm in length in a vertical direction A along the second terminal portion 46 and the fourth terminal portion 48.

In this preferred embodiment, the pair of application electrodes 39A, 39B are each formed in a substantially rectangular shape which is vertically long. The application electrodes 39A, 39B have respectively first terminal portions 86A, 86B, second terminal portions 87A, 87B, third terminal portions 88A, 88B and fourth terminal portions 89A, 89B, each of which constitutes a side of the rectangular shape.

The first terminal portions 86A, 86B are, for example, upper terminal portions of the respective application electrodes 39A, 39B when each of the application electrodes 39A, 39B is attached to the human body 1 and opposed to the third terminal portions 88A, 88B. That is, the third terminal portions 88A, 88B are lower terminal portions of the respective application electrodes 39A, 39B when each of the application electrodes 39A, 39B is attached to the human body 1. The second terminal portions 87A, 87B and the fourth terminal portions 89A, 89B couple the first terminal portions 86A, 86B and the third terminal portions 88A, 88B and are opposed to each other.

Each of the application electrodes 39A, 39B is, for example, about 5.3 cm in length in the horizontal direction B along the first terminal portions 86A, 86B and the third terminal portions 88A, 88B and about 9.5 cm in length in the vertical direction A along the second terminal portions 87A, 87B and the fourth terminal portions 89A, 89B. That is, a total length of the pair of application electrodes 39A, 39B in the horizontal direction B is longer than the length of the indifferent electrode 38 in the horizontal direction B.

A first terminal 90 is integrally provided on the second surface 41 of the indifferent electrode 38. The first terminal 90 protrudes from the second surface 41 of the indifferent electrode 38. The first terminal 90 has a first socket 91 which faces one side (upper side in FIG. 5) and is formed in a tubular shape in which the other side (lower side in FIG. 5) is closed. In this preferred embodiment, the first socket 91 is flush with the first terminal portion 45 of the indifferent electrode 38.

Second terminals 92A, 92B are integrally provided on the respective second surfaces 43A, 43B of the pair of application electrodes 39A, 39B. The second terminals 92A, 92B protrude from the second surfaces 43A, 43B of the pair of application electrodes 39A, 39B. The second terminals 92A, 92B respectively have second sockets 93A, 93B which face in the same direction as the first socket 91 and are each formed in a tubular shape in which the other side (lower side in FIG. 5) is closed. In this preferred embodiment, the second sockets 93A, 93B are respectively flush with the first terminal portions 86A, 86B of the pair of application electrodes 39A, 39B.

Further, a thin portion 94 is formed on the second surface 41 of the indifferent electrode 38. The thin portion 94 is a section which is formed so as to be relatively thin in the indifferent electrode 38 and has, for example, a thickness of 0.3 mm to 2.0 mm. The thin portion 94 includes a pair of thin portions 94 which are linear regions (having a width of, for example, about 53 mm) along the second terminal portion 46 and the fourth terminal portion 48.

The pair of thin portions 94 extend in parallel to each other from the first terminal portion 45 of the indifferent electrode 38 up to the third terminal portion 47 thereof and are also disposed, with the first terminal 90 held between them. The pair of thin portions 94 are both separated from the first terminal 90 in the direction B along the first terminal portion 45 and the third terminal portion 47. By the pair of thin portions 94 being formed, by which the indifferent electrode 38 is formed so as to be easily folded, with the thin portions 94 used as folds. Thereby, the indifferent electrode 38 can be favorably attached in accordance with the curvature of the skin of the human body 1.

Thin portions 95A, 95B are formed on the second surfaces 43A, 43B of the respective application electrodes 39A, 39B. The thin portion 95A, 95B are sections which are formed relatively thin in the respective application electrodes 39A, 39B, having a thickness of, for example, 0.3 mm to 2.0 mm. The thin portions 95A, 95B respectively include a plurality of thin portions 95A, 95B which are linear regions (having a width of, for example, about 53 mm) extending from the terminal portions of the respective application electrodes 39A, 39B (for example, the second terminal portions 87A, 87B of the application electrodes 39A, 39B and the fourth terminal portions 89A, 89B of the application electrodes 39A, 39B) to the third terminal portions 88A, 88B.

The plurality of thin portion 95A, 95B extend in parallel to each other from the second terminal portions 87A, 87B of the application electrodes 39A, 39B and the fourth terminal portions 89A, 89B of the application electrodes 39A, 39B to the third terminal portions 88A, 88B. In this preferred embodiment, the three thin portions 95A and 95B are formed respectively in a striped pattern.

Each of the application electrodes 39A, 39B is formed so as to be easily folded, with the thin portions 95A, 95B given as folds, due to formation of the plurality of thin portions 95A, 95B. Thereby, each of the application electrodes 39A, 39B can be favorably attached to the skin in accordance with the curvature of the skin of the human body 1. Further, linear thin portions 95A, 95B are formed which connect the terminal portions adjacent to each other, with corner portions 96A, 96B of the respective application electrodes 39A, 39B given as boundaries. Further, in this preferred embodiment, they are formed sequentially in a striped pattern from the corner portions 96A, 96B toward an inner region. Therefore, for example, after treatment, the corner portions 96A, 96B are picked up with fingers, thus making it possible to easily peel off the respective application electrodes 39A, 39B from the corner portions 96A, 96B.

Then, the indifferent electrode 38 and the pair of application electrodes 39A, 39B are each arranged with a conductive rubber sheet which is composed of a rubber base 44 and a conductive sheet 97 embedded in the rubber base 44.

The rubber base 44 forms an external shape of each of the indifferent electrode 38 and the pair of application electrodes 39A, 39B. On the other hand, the conductive sheet 97 is covered with the rubber base 44 and thereby embedded in the rubber base 44. In FIG. 5 and FIG. 6, a region in which the conductive sheet 97 is embedded is shown by a dotted line in each of the indifferent electrode 38 and the pair of application electrodes 39A, 39B.

In this preferred embodiment. the rubber base 44 is arranged with a sheet which is composed of silicon rubber that contains carbon black. A material of the rubber base 44 is not limited to silicon rubber that contains carbon black, as long as it is a conductive rubber. For example, a conductor (conductive filler) which is mixed with silicon rubber may include a silver powder, silica and graphite that are gold-plated, conductive zinc oxide in addition to carbon black. An ion conductive silicon rubber may also be used as a material of the rubber base 44.

In this preferred embodiment, the conductive sheet 97 is arranged with a conductive mesh. As the conductive mesh, for example, a mesh formed with conductive fibers such as silver threads is included. As shown in FIG. 7, the conductive sheet 97 has a number of openings 49 (lattice window section) in its plane.

The conductive sheet 97 is embedded in the sheet-like rubber base 44 substantially in its entirety. Here, "substantially in its entirety" means that, between a periphery of the conductive sheet 97 and a periphery of the rubber base 44 (in this preferred embodiment, the respective terminal portions 45 to 48 and 86A, 86B to 89A, 89B of the indifferent electrode 38 and the pair of application electrodes 39A, 39B), there may be provided a small margin (a section 98 which is exclusively composed of the rubber base 44 in its entire thickness direction). In this preferred embodiment, an entire circumference of the conductive sheet 97 is surrounded by the section 98 of the rubber base 44. The margin may have a size which is set, for example, with a positional deviation of the conductive sheet 97 at the time of production taken into account.

Therefore, as shown in FIG. 5, the conductive sheet 97 may overlap with the first terminal 90 and the second terminals 92A, 92B provided at the respective first terminal portions 45, 86A, 86B of the indifferent electrode 38 and the pair of application electrodes 39A, 39B. In other words, in the indifferent electrode 38 and the pair of application electrodes 39A, 39B, the conductive sheet 97 may be embedded in the first terminal 90 and the second terminals 92A, 92B.

Further, in this preferred embodiment, the conductive sheet 97 is disposed biased toward the sides of the second surfaces 41, 43A, 43B of the rubber base 44 (surfaces not in contact with the skin of the human body 1) in a thickness direction of the rubber base 44. Thereby, a thickness T₁ from the conductive sheet 97 to the first surfaces 40, 42A, 42B of the rubber base 44 (surfaces in contact with the skin of the human body 1) is compared with a thickness T₂ from the conductive sheet 97 to the second surfaces 41, 43A, 43B of the rubber base 44 to find that the thickness T₁ is thicker than the thickness T₂.

That is, the indifferent electrode 38 and the pair of application electrodes 39A, 39B may have a first section 99 of the rubber base 44 having a relatively large thickness T₁, the conductive sheet 97 and a second section 100 of the rubber base 44 having a relatively small thickness T₂ sequentially from the sides of the first surfaces 40, 42A, 42B. In other words, the indifferent electrode 38 and the pair of application electrodes 39A, 39B may have a three-layer structure composed of a first rubber layer 99 having a relatively large thickness T₁, the conductive sheet 97 and a second rubber layer 100 having a relatively smaller thickness T₂ sequentially from the sides of the first surfaces 40, 42A, 42B.

In manufacturing the above-described electrode pad 37, for example, first, a rubber sheet is prepared as a material of the conductive sheet 97 and the rubber base 44. Next, a mold is heated in advance up to a predetermined temperature that is equal to or higher than a temperature at which the rubber sheet becomes soft and, thereafter, the conductive sheet 97 and the rubber sheet are stacked in this order inside the mold. Next, a surface of the rubber sheet is pressed, by which the conductive sheet 97 and the rubber sheet are press-molded. Thereby, the softened material of the rubber sheet spreads up to a shape of the mold and also spreads around both the front and rear surfaces of the conductive sheet 97 through the openings 49 of the conductive sheet 97. As a result, the conductive sheet 97 is in such a state that it is embedded into the material of the rubber sheet having the shape of the rubber base 44. Thereafter, the mold is cooled to remove the rubber base 44 from the mold, thereby providing the indifferent electrode 38 and the pair of application electrodes 39A, 39B.

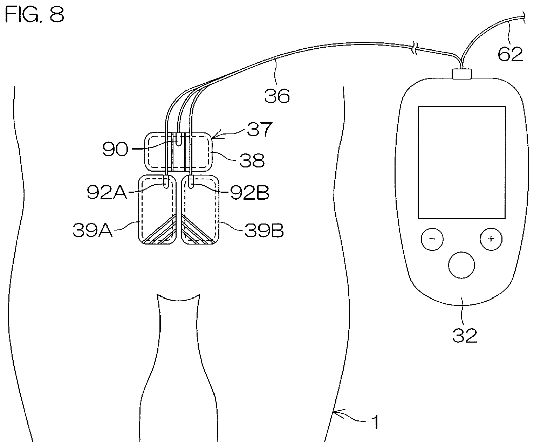

Then, in order to attach the electrode pad 37 to the human body 1, as shown in FIG. 8, a wiring plug (not shown) which is connected to a leading end of the wiring 36 is connected to the first terminal 90 and the second terminals 92A, 92B, and the electrode pad may be attached to the skin directly at the back of the sacral bone of a person to be treated by way of a separately prepared conductive/adhesive pad (for example, a conductive/adhesive gel pad, etc.).

On the other hand, the toe electrode 61 may include a first electrode 63, a second electrode 64 and a third electrode 65. In this preferred embodiment, the third electrode 65 may be a reference electrode, and the first electrode 63 may be an electrode (negative electrode) having a negative potential with respect to the third electrode 65. The second electrode 64 may be an electrode (positive electrode) having a positive potential with respect to the third electrode 65.

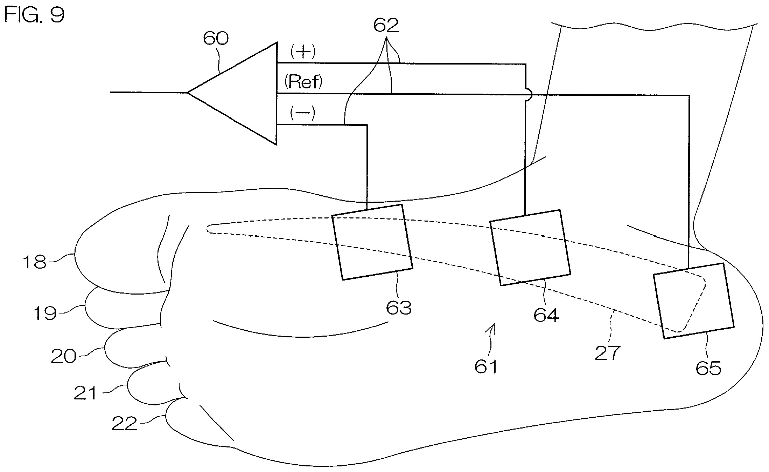

Then, in order to attach the toe electrode 61 to the human body 1, as shown in FIG. 9, the first electrode 63, the second electrode 64 and the third electrode 65 may be attached in this order from a tiptoe side, for example, along a direction in which muscle fibers of an abductor hallucis muscle 27 of a foot extend so as to oppose the muscle fibers of the abductor hallucis muscle 27. The abductor hallucis muscle 27 is a muscle fiber which is controlled by a nerve connected to the peroneal nerve 16 and the tibial nerve 17 (for example, medial plantar nerves (L5~S2)). Further, it is preferable that the first electrode 63 and the second electrode 64 are attached so as to widen the distance between the electrodes, for example, by attaching the first electrode 63 to a base of the big toe (ball of the foot) and attaching the second electrode 64 to an intermediate site between the heel and the base of the big toe (ball of the foot).

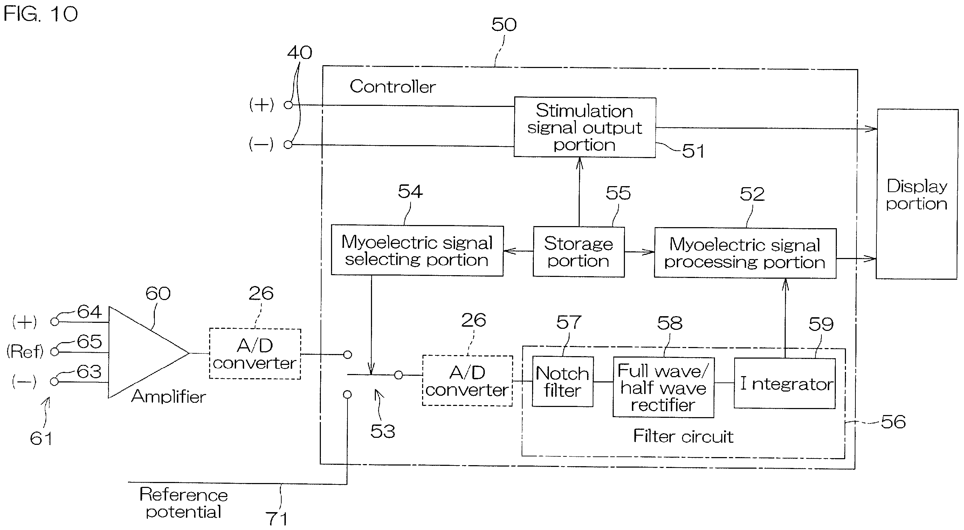

FIG. 10 is a block diagram which shows an electric configuration of the electrical stimulation therapeutic device 31.

A wiring substrate (not shown) is built in the casing 32 of the electrical stimulation therapeutic device 31, and a controller 50 is provided in the wiring substrate. The monitor 33 (display portion), the start/stop button 34 described above, the operation button 35 (input portion), the pair of application electrodes 39 and the toe electrode 61 are electrically connected to the controller 50. An input signal from the start/stop button 34 and the operation button 35 is input to the controller 50, and an output signal from the controller 50 is output to the pair of application electrodes 39. Further, a myoelectric signal detected by the toe electrode 61 is input to the controller 50 and processed by the controller 50.

The controller 50 may be arranged with a semiconductor chip. In this preferred embodiment, the controller 50 includes a stimulation signal output portion 51, a myoelectric signal processing portion 52, a switch portion 53, a myoelectric signal selecting portion 54, a storage portion 55 and a filter circuit 56.

The stimulation signal output portion 51 may be arranged with a semiconductor integrated circuit (IC: Integrated Circuit), for example, a CPU, memory such as ROM and RAM, a timer and the like. The stimulation signal output portion 51 may be referred to as a stimulation signal output circuit. The stimulation signal output portion 51 outputs a stimulation voltage stored in advance in the storage portion 55 to the pair of application electrodes 39. Information on the stimulation voltage which is stored in the storage portion 55 includes, for example, a width of a pulse wave (pulse width) of the stimulation signal, a frequency and the like which are suitable for each of the persons to be treated.

Further, the stimulation signal output portion 51 displays the information on an electrical stimulation given to a person to be treated (pulse width, frequency, etc.) on the monitor 33. In this preferred embodiment, a magnitude of a pulse amplitude of the electrical stimulation may be demarcated in advance in a stepwise manner into a plurality of stages (for example, five stages) and may display on the monitor 33 the stage of the amplitude concerned depending on a magnitude of the output amplitude.

In FIG. 10, the stimulation signal output portion 51 is shown as a configuration independent from the storage portion 55. However, it may have a built-in section which stores information with regard to the stimulation signal output portion 51, for example, among the information stored in the storage portion 55.

The myoelectric signal processing portion 52 may be arranged with a semiconductor integrated circuit (IC: Integrated Circuit) including, for example, a CPU, memory such as ROM, RAM, a timer and the like. The myoelectric signal processing portion 52 may be referred to as a myoelectric signal processing circuit. The myoelectric signal processing portion 52 processes a myoelectric signal detected by the toe electrode 61 and displays it on the monitor 33. For example, the myoelectric signal processing portion 52 may quantitatively calculate an amplitude of the myoelectric signal detected by the toe electrode 61 and may visually display the result on the monitor 33. In this preferred embodiment, a magnitude of the amplitude may be in advance demarcated in a stepwise manner into a plurality of stages (for example, five stages) and may display on the monitor 33 the stage of the amplitude concerned depending on a magnitude of the calculated amplitude.

Further, the myoelectric signal processing portion 52 is designed so as not to detect and process a myoelectric signal under a condition that is stored in advance in the storage portion 55. In this preferred embodiment, the myoelectric signal is not detected or processed during a detection stop period of time 25 (to be described later) which is set on the basis of a quotient (x/v) obtained when a distance (x) from the sacral bone 3 of the human body 1 to surfaces of the toes 18 to 22 is divided by a transmission velocity (v) of the nerve S3, etc., which pass thorough the sacral bone 3 of the human body 1 or the vicinity of the sacral bone 3.

In FIG. 10, the myoelectric signal processing portion 52 is shown as a configuration independent from the storage portion 55. However, it may have a built-in section which stores information with regard to the myoelectric signal processing portion 52, for example, among the information stored in the storage portion 55.

The switch portion 53 turns on or off a connection of the toe electrode 61 (detection electrode) with the myoelectric signal processing portion 52. The switch portion 53 may be arranged, for example, with an analog switch (for example, an analog switch arranged with a CMOS, a bipolar transistor, a relay, etc.) or may be arranged so as to be operated partially or entirely by digital signal processing.

The myoelectric signal selecting portion 54 may be arranged with a semiconductor integrated circuit (IC: Integrated Circuit) including, for example, a CPU, memory such as ROM and RAM, a timer and the like. The myoelectric signal selecting portion 54 may be referred to as a myoelectric signal selecting circuit. The myoelectric signal selecting portion 54 controls the switch portion 53 so that, during the detection stop period of time 25 (to be described later) set in the storage portion 55, a connection of the toe electrode 61 with the myoelectric signal processing portion 52 can be turned off and also, at the end of the detection stop period of time 25, the connection of the toe electrode 61 with the myoelectric signal processing portion 52 can be turned on.

In FIG. 10, the myoelectric signal selecting portion 54 is shown as a configuration independent from the storage portion 55. However, it may have a built-in section which stores information with regard to the myoelectric signal selecting portion 54, for example, among the information stored in the storage portion 55.

The storage portion 55 is arranged, for example, with memory such as ROM and RAM. The storage portion 55 stores, for example, information on the stimulation voltage, the detection stop period of time 25 and the like.

In this preferred embodiment, the filter circuit 56 includes a notch filter 57, a full wave/half wave rectifier 58 and an integrator 59. Thereby, it is possible to lower noise of the myoelectric signal input to the myoelectric signal processing portion 52. The integrator 59 is also provided to easily detect an amplitude, a peak, etc., of the myoelectric signal, thus making it possible to process efficiently the myoelectric signal in the myoelectric signal processing portion 52. The integrator 59 may be referred to as an integration circuit.

The filter circuit 56 is not limited to the configuration of FIG. 10 but may have a configuration which is adopted in a known filter circuit. For example, in place of the notch filter 57, a low pass filter, a high pass filter, a band pass filter or others may be adopted.

Further, the filter circuit 56 may be entirely arranged with an analog circuit or may be configured so as to be operated partially or entirely by digital signal processing.

Also, where the filter circuit 56 is entirely arranged with an analog circuit, in order to further lower noise, the previously described analog circuit section may have a block configuration which is insulated from the other digital circuits.

An amplifier 60 for amplifying the myoelectric signal detected by the toe electrode 61 is also provided between the toe electrode 61 and the switch portion 53.

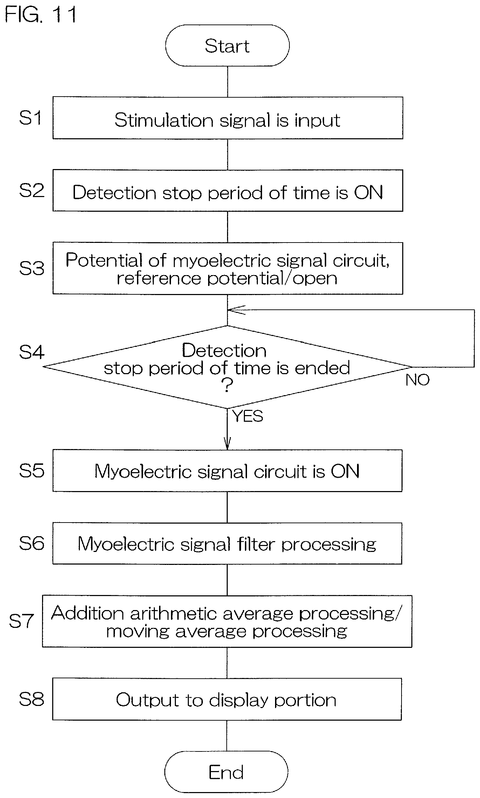

FIG. 11 is a flowchart of monitoring of myoelectric data of the electrical stimulation therapeutic device 31.

In order to give treatment by using the electrical stimulation therapeutic device 31, for example, as shown in FIG. 8, a person to be treated first attaches the electrode pad 37 to his/her body.

After the electrode pad 37 has been attached, the operation button 35 is operated to select a treatment menu suitable for the person to be treated and the start/stop button 34 is depressed. Thereby, an electrical stimulation signal is output from the electrode pad 37 to stimulate the third sacral nerve S3, by which treatment by the electrical stimulation therapeutic device 31 can be started (Step S1). A condition of the stimulation signal (output pulse) is, for example, a pulse width of 1 µs (microsecond) to 500 µs (microseconds). The output pulse is continuously output at a frequency of 1Hz to 50Hz to constitute a stimulation signal pulse 66 in which one cycle is given as T.

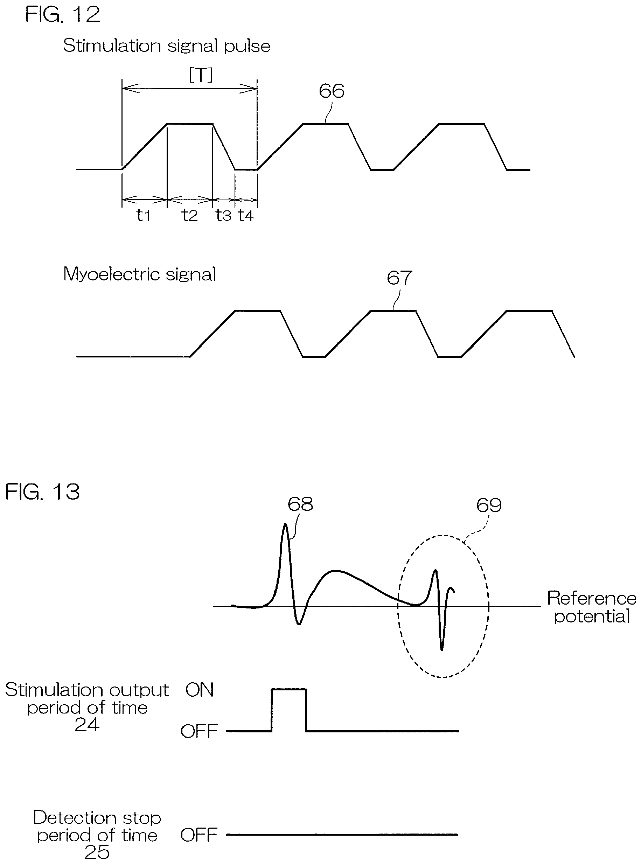

More specifically, as shown in FIG. 12, the stimulation signal pulse 66 may be continuously applied at a pattern of six seconds in total consisting of a rise section t₁ = 2 seconds, a continuation section t₂ = 2 seconds, a fall section t₃ = 1 second and an interval until a next pulse t₄ = 1 second. As a matter of course, t₁, t₂, t₃ and t₄ can be changed in length and a voltage can be changed in magnitude, whenever necessary, according to the body size of a user. For example, the rise section t₁ may be set at the same time as the fall section t₃.

On the other hand, the toe electrode 61 may be attached to a toe as a detection electrode for confirming (monitoring) whether a stimulation signal from the electrode pad 37 is appropriately transmitted to the pelvic nerve 13 and the pudic nerve 14. The toe to which the toe electrode 61 is attached may be any one of a first toe 18 to a fifth toe 22, and the first toe 18 is preferable in view of easy attachment.

More specifically, as shown in FIG. 9, the first electrode 63, the second electrode 64 and the third electrode 65 may be attached in this order from the tiptoe side, for example, along a direction in which muscle fibers of the abductor hallucis muscle 27 of a foot extend so as to oppose the muscle fibers of the abductor hallucis muscle 27.

Then, measurement data (for example, 40 to 60 pulse portions) obtained from muscular contraction in association with a stimulation from the electrode pad 37 is averaged to remove noise and the like, by which a myoelectric data waveform (waveform of a myoelectric signal 67 in FIG. 12) is prepared. Then, the waveform of the myoelectric signal 67 is compared with a waveform of the stimulation signal pulse 66. Where the waveform of the myoelectric signal 67 is generated in synchronization with the waveform of the stimulation signal pulse 66, it means that the pair of application electrodes 39 are attached at an appropriate position to give an electrical stimulation.

Here, as shown in FIG. 13, a consideration is given to a case where the myoelectric signal processing portion 52 continues to detect and process a myoelectric signal detected by the toe electrode 61 from the start of outputting a stimulation (start of the electrical stimulation by the pair of application electrodes 39). That is, it is a case where, during a stimulation output period of time 24, there is not provided the detection stop period of time 25 during which the myoelectric signal processing portion 52 does not detect and process the myoelectric signal (the detection stop period of time 25 is turned off) .

In this case, when an electrical stimulation is given from the pair of application electrodes 39 to the back of the sacral bone 3 of the human body 1, a stimulation voltage is transmitted to the toes 18 to 22 as noise, thereby generating a large noise 68 in a superimposed manner in myoelectric data of the toes 18 to 22. Therefore, there is a case that an electromyogram portion 69 which corresponds to the waveform of the myoelectric signal 67 in FIG. 12 and is necessary for myoelectric monitoring may not be accurately detected.

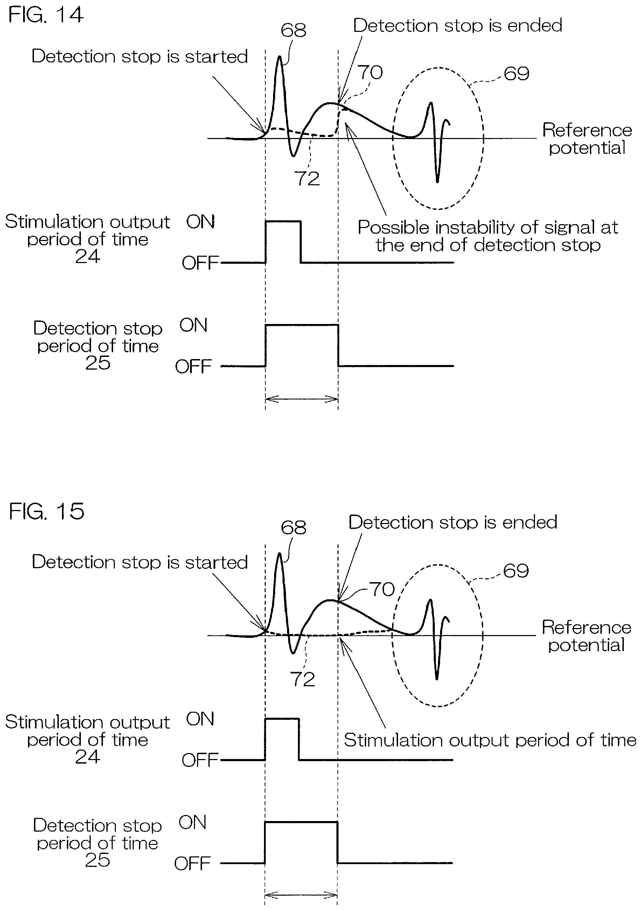

Thus, in this preferred embodiment, as shown in FIG. 14, for a certain period of time including the stimulation output period of time 24 from the start of outputting a stimulation, the detection stop period of time 25 during which the myoelectric signal processing portion 52 does not detect and process a myoelectric signal is turned on (Step S2). Thereby, the large noise 68 generated at the start of outputting the stimulation can be eliminated and during the detection stop period of time 25, a myoelectric signal 72 can be provided, the waveform of which is lower in noise. In this respect, in this preferred embodiment, the detection stop period of time 25 is set on the basis of a quotient (x/v) obtained when a distance (x) from the sacral bone 3 of the human body 1 to surfaces of the toes 18 to 22 is divided by a transmission velocity (v) of the nerve S3, etc., which pass through the sacral bone 3 of the human body 1 or the vicinity of the sacral bone 3. That is, during the detection stop period of time 25, the myoelectric signal of the toes 18 to 22 is not detected in the myoelectric signal processing portion 52.

The stimulation signal given to the human body 1 during the detection stop period of time 25 does not reach the toes 18 to 22 by way of the nerve S3, etc. That is, the myoelectric signal detected for a time during which the stimulation signal thereof reaches the toes 18 to 22 by way of the nerve S3, etc., after application of an electrical stimulation is not a signal which carries information necessary for myoelectric monitoring but the noise 68. Therefore, during the detection stop period of time 25, the myoelectric signal of the toes 18 to 22 is not detected in the myoelectric signal processing portion 52, thus making it possible to eliminate the noise 68 of the myoelectric signal. Thereby, it is possible to accurately detect the electromyogram portion 69 which reaches the toes 18 to 22 by way of the nerve S3, etc., which pass through the sacral bone 3 or the vicinity of the sacral bone 3 and is necessary for myoelectric monitoring.

In this preferred embodiment, the detection stop period of time 25 is given as 25 ms, for example. However, the detection stop period of time 25 can be set at 15 ms to 25 ms, for example. As a matter of course, the detection stop period of time 25 is not limited to the above range but may be determined by referring to sample data covering age and gender of patients on the basis of several patterns on a distance from the sacral bone of a human body to a surface of a toe as well as a transmission velocity of a nerve which passes through the sacral bone of the human body and the vicinity of the sacral bone.

As shown in FIG. 14, the detection stop period of time 25 is only provided, thus making it possible to improve accuracy of the myoelectric monitoring. However, control subsequent to Step S3 in FIG. 11 can be conducted to further improve the accuracy of the myoelectric monitoring.

That is, in FIG. 14, although no myoelectric signal is detected in the myoelectric signal processing portion 52 during the detection stop period of time 25, the myoelectric signal processing portion 52 is continuously connected with the toe electrode 61 (amplifier 60) by way of the filter circuit 56. Therefore, a potential of the myoelectric signal input to the myoelectric signal processing portion 52 is changed over time. Therefore, at the end of the detection stop period of time 25, a section 70 which appears to be a peak occurs in a waveform of the myoelectric signal 72 due to influences of a time constant of the filter circuit 56, etc., and the myoelectric signal 72 at the end thereof may become slightly unstable.

Thus, in this preferred embodiment, at the start of the detection stop period of time 25, a connection of the toe electrode 61 with the myoelectric signal processing portion 52 is turned off. More specifically, the myoelectric signal selecting portion 54 makes a potential of the switch portion 53 into a reference potential 71 or a pre-held drift potential or makes the switch portion 53 into an open state (open) on the basis of output of the stimulation signal (start of the detection stop period of time 25) (Step S3). Thereby, in relation to the switch portion 53, a potential of the myoelectric signal processing portion 52 is fixed to the reference potential 71 or the pre-held drift potential or fixed to a potential at the time of input of the stimulation signal (at the start of the detection stop period of time 25). The reference potential 71 is, for example, a neutral voltage which is an operation reference point of the amplifier 60, and a neutral voltage of 0 volts of a ± power source, a neutral voltage which is an operation reference point of a circuit used in the filter circuit 56, a voltage when the myoelectric signal is not amplified by output of the amplifier 60 or a ground potential, etc., may be set as the reference potential.

Thereafter, a determination is made for whether the detection stop period of time 25 is ended (Step S4). When the detection stop period of time 25 is ended (YES in Step S4), the switch portion 53 is switched by the myoelectric signal selecting portion 54, by which a connection of the toe electrode 61 with the myoelectric signal processing portion 52 is turned on (Step S5). As described above, the connection of the toe electrode 61 with the myoelectric signal processing portion 52 is turned off at the start of the detection stop period of time 25 and turned on at the end of the detection stop period of time 25. Thereby, as shown in FIG. 15, the section 70 which appears to be a peak does not occur at the end of the detection stop period of time 25, and a potential of the myoelectric signal 72 can be stabilized.

Then, in the filter circuit 56, the myoelectric signal is filtered by passing through the notch filter 57 and the full wave/half wave rectifier 58, and a signal set is produced by the integrator 59 (Step S6). More specifically, the plurality of myoelectric signals 73 detected by the toe electrode 61 are integrated for a predetermined sampling time and output to the myoelectric signal processing portion 52 as a signal set 74 (first signal) which has been integrated. The sampling time may be, for example, for a stimulation output period of time t₁ + t₂ + t₃ seconds to T seconds.

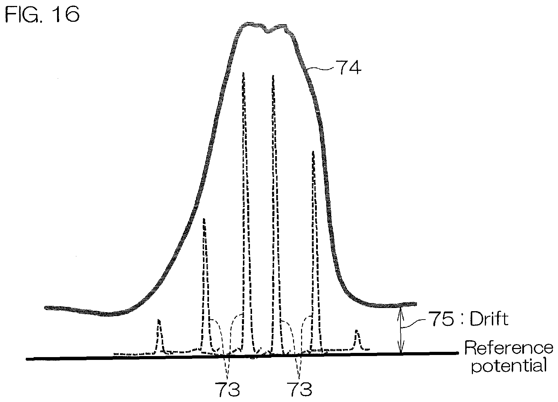

Here, as shown in FIG. 16, during treatment by the electrical stimulation therapeutic device 31, a change in reference line (drift 75) may be found in an electromyogram due to a change arising from movements of a human body 1, etc., a change by introduction of a commercial alternating current, introduction of noise from an electric appliance around the human body 1 (for example, a smart phone, etc.) and the like.

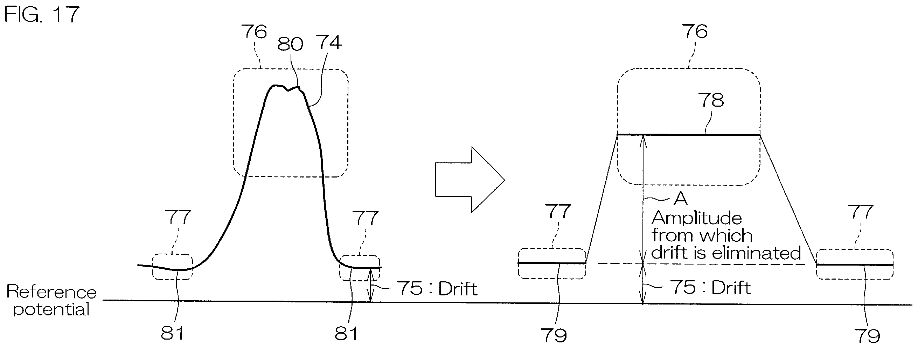

Thus, in the myoelectric signal processing portion 52, an amplitude of the signal set 74 is calculated on the basis of a difference between an averaged value of the first section 76 of the signal set 74 for a predetermined first period of time and an averaged value of the second section 77 of the signal set 74 for a predetermined second period of time temporally before and after the first section 76. More specifically, the first section 76 and the second section 77 of the signal set 74 are averaged by being subjected to addition arithmetic average processing or moving average processing (Step S7). Thereby, as shown in FIG. 17, an averaged value of the first section 76 (first average value 78) and an averaged value of the second section 77 (second average value 79) are calculated.

The first section 76 may be set as a period of time which includes, for example, ± t₂ seconds before and after a peak portion 80 of the signal set 74. On the other hand, the second section 77 may be set as a period of time which is 0 seconds to t₄ seconds of a foot portion 81 of the signal set 74.

Then, the second average value 79 is subtracted from the first average value 78, thus making it possible to eliminate the drift 75 of the signal set 74. As a result, it is possible to accurately detect an amplitude A, a peak, etc., of the signal set 74. Where a signal above a certain level is input in the second section 77, based on a judgment that a normal myoelectric signal is not obtained, an error message may be displayed on the monitor 33 (for example, a second level gauge 83 to be described later is lit in a color different from a normal color).

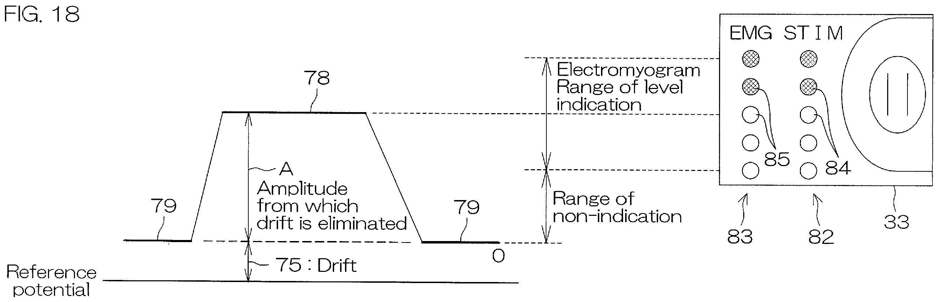

Next, a strength of the processed signal set 74 is output on the monitor 33 (display portion) (Step S8). In this preferred embodiment, first, as shown in FIG. 18, a first level gauge 82 which shows a strength of stimulation output (STIM: stimulation) and a second level gauge 83 which shows a strength of the myoelectric signal (electromyogram EMG: electromyography) are displayed on the monitor 33.

In this preferred embodiment, the first level gauge 82 is demarcated into five stages. Depending on a magnitude of an amplitude of an output stimulation pulse, individual lighting portions 84 of the first level gauge 82 are lit sequentially from below. When the first level gauge 82 is deflected in such a manner that all five lighting portions 84 can be lit, it means that an amplitude of the stimulation pulse is a maximum.

Similarly, in this preferred embodiment, the second level gauge 83 is demarcated into five stages. Depending on a magnitude of an amplitude A of the myoelectric signal calculated by the myoelectric signal processing portion 52, individual lighting portions 85 of the second level gauge 83 are lit sequentially from below. When the second level gauge 83 is deflected in such a manner so that all five lighting portions 85 can be lit, it means that the myoelectric signal is a maximum.

Then, in this preferred embodiment, as shown in FIG. 12, where the pair of application electrodes 39 are attached at an appropriate position to give treatment, a waveform of the myoelectric signal 67 is generated in synchronization with an interval between waveforms of the stimulation signal pulse 66. Therefore, in the monitor 33 of FIG. 18, first, after the first level gauge 82 has been deflected (after lighting is turned off at a time when the lighting portions 84 are lit sequentially from below and lighting reaches up to the lighting portion 84 which corresponds to a particular magnitude of the stimulation pulse), the second level gauge 83 is deflected. Where a level indication of the second level gauge 83 is deflected in synchronization with a level indication of the first level gauge 82, it means that the pair of application electrodes 39 are attached at an appropriate position to give an electrical stimulation.

For example, the above case is such that the level indication of the first level gauge 82 and the level indication of the second level gauge 83 are made alternately at a predetermined interval. Therefore, a person to be treated and medical personnel only visually observe the first level gauge 82 and the second level gauge 83 of the monitor 33, thus making it possible to easily confirm whether the pair of application electrodes 39 are attached at an appropriate position. Where the pair of application electrodes 39 are determined not to be attached at an appropriate position on the basis of the confirmation, supply of the stimulation signal from the pair of application electrodes 39 may be stopped.

Further, in this preferred embodiment, even if an amplitude A from which the drift 75 has been eliminated exceeds zero, it is not displayed on the second level gauge 83 if the amplitude A is less than a predetermined magnitude. Thereby, the myoelectric signal having the amplitude A which is less than a predetermined magnitude is not displayed and the myoelectric signal arising from an unnecessary noise can be prevented from being displayed on the second level gauge 83. As a result, it is possible to easily confirm a relationship between the stimulation signal and the myoelectric signal generated in response thereto.

A description has been so far given of the preferred embodiments of the present invention. However, the present invention can be carried out in other modes.

For example, in the previously described preferred embodiment, the urination disorder treatment device (fecal incontinence treatment device) is adopted as an example of the electrical stimulation therapeutic device. However, the present invention is not limited to the urination disorder treatment device or the fecal incontinence treatment device but can be applied to devices in general which are used in electrical stimulation therapy for diseases other than the above diseases.

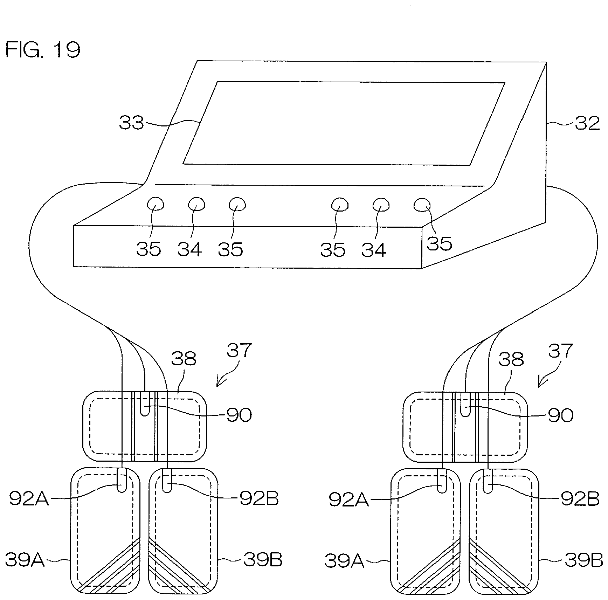

Further, in the previously described preferred embodiment, a description has been given of a configuration of the portable-type electrical stimulation therapeutic device 31. However, as shown in FIG. 19, an electric configuration of the electrical stimulation therapeutic device 31 and control thereof may be applied to a stationary type (installation type) electrical stimulation therapeutic device 23. The above-described stationary-type electrical stimulation therapeutic device 23 is commonly used by a plurality of patients in a medical institution. Therefore, it may be provided with a memory which stores past treatment data of each patient.

Further, for example, in the previously described preferred embodiment, during the detection stop period of time 25, no myoelectric signal is to be detected by operation inside the myoelectric signal processing portion 52. In contrast thereto, where a configuration is made by subjecting the switch portion 53 or the filter circuit 56 to digital signal processing, for example, as shown in FIG. 10, the A/D converter 26 may be provided between the toe electrode 61 and the switch portion 53 or between the switch portion 53 and the filter circuit 56. Thereby, analog/digital conversion by the A/D converter 26 is stopped during the detection stop period of time 25, thus making it possible to stop detection of the myoelectric signal by the myoelectric signal processing portion 52.

There may be provided such a configuration that, during a sampling time, sampling is not conducted constantly but, for example, sampling is conducted only during a time of continuation section t₂ even during the sampling time. In this case, the detection stop period of time 25 is not given as a fixed value but made sufficiently large so as not to be detected constantly in the rise section t₁ or the fall section t₃, that is, the plurality of detection stop periods of time 25 are used, by which the above configuration can be realized easily.

Further, in this preferred embodiment, the detection signal is subjected to sampling for each of individual output pulses which constitute a stimulation signal pulse. However, without limiting thereto, the detection signal may be subjected to sampling for each of the specific number of output pulses, for example, every two pulses or every three pulses. In this case as well, the plurality of detection stop periods of time 25 are prepared and one detection stop period of time 25 is, for example, set for a portion of two output pulses, etc., by which the sampling can be easily realized. The above processing is effective in a case where there is found a limitation on a usable memory capacity or a processing ability of a CPU upon processing of the above drift or processing of the amplitude A by using digital data.

Further, for example, as a means for confirming whether the pair of application electrodes 39 are attached at an appropriate position, in place of the level indications of the first level gauge 82 and the second level gauge 83, etc., in the previously described preferred embodiment, as shown in FIG. 12, there may be adopted a method for displaying a waveform of a signal on the monitor 33.

Further, in the previously described preferred embodiment, for example, as an example of the display portion of the present invention, the liquid crystal monitor 33 which displays a message and an image, etc., for a user is shown. However, a means that displays operating conditions of the electrical stimulation therapeutic device 31 for a user may not be necessarily the monitor 33. For example, messages to a person to be treated (such as an error message, the first level gauge 82 and the second level gauge 83) are in advance provided on a front panel of the casing 32, an LED or others may be lit or a lamp in the vicinity of the message concerned may be lit, thereby notifying a person to be treated.

Further, in the previously described preferred embodiment, there is shown an example for efficiently detecting the electromyogram portion 69. However, the noise 68 arising from the stimulation signal may be isolated from the electromyogram portion 69 so that the electromyogram portion 69 can only be detected without fail. For example, an amplitude level of the noise 68 during the detection stop period of time 25 is processed so as to be much smaller than the electromyogram portion 69 (for example, processing for reducing the amplitude to 1/100), thus making it possible to detect exactly only the myoelectric signal after an elapse of the detection stop period of time 25.

In addition, the design of the present invention may be modified in various ways without departing from the scope described in the clams.

The present application corresponds to Japanese Patent Application No. 2019-173397 filed in the Japan Patent Office on September 24, 2019, and the entire disclosure of this application is incorporated herein by reference.

### Reference Signs List

- 1: Human body
- 3: Sacral bone
- 12: Hypogastric nerve
- 13: Pelvic nerve
- 14: Pudic nerve
- 15: Ischiadic nerve
- 16: Peroneal nerve
- 17: Tibial nerve
- 18: First toe (big toe)
- 19: Second toe
- 20: Third toe
- 21: Fourth toe
- 22: Fifth toe (little toe)
- 23: Electrical stimulation therapeutic device
- 25: Detection stop period of time
- 31: Electrical stimulation therapeutic device
- 33: Monitor
- 37: Electrode pad
- 39: Application electrode
- 50: Controller
- 51: Stimulation signal output portion
- 52: Myoelectric signal processing portion
- 53: Switch portion
- 54: Myoelectric signal selecting portion
- 55: Storage portion
- 56: Filter circuit
- 57: Notch filter
- 58: Full wave/half wave rectifier
- 59: Integrator
- 60: Amplifier
- 61: Toe electrode
- 62: Toe wiring
- 63: First electrode
- 64: Second electrode
- 65: Third electrode
- 66: Stimulation signal pulse
- 67: Myoelectric signal
- 68: Noise
- 69: Electromyogram portion
- 71: Reference potential
- 74: Signal set
- 76: First section
- 77: Second section
- 78: First average value
- 79: Second average value
- 82: First level gauge
- 83: Second level gauge
- A: Amplitude

## Claims

1. An electrical stimulation therapeutic device comprising:
a pair of application electrodes which are disposed at the back of a sacral bone of a person to be treated and supplies an electrical stimulation signal from the back of the sacral bone;
a detection electrode which is disposed on a surface of a toe of a person to be treated and detects a myoelectric signal of the toe;
a display portion which determines whether the myoelectric signal of the toe is generated in response to the stimulation signal; and
a myoelectric signal processing portion which processes the myoelectric signal detected by the detection electrode and displays it visually on the display portion; wherein
the myoelectric signal processing portion does not detect the myoelectric signal of the toe during a predetermined detection stop period of time from output of the stimulation signal and
the detection stop period of time is set on the basis of a quotient (x/v) obtained when a distance (x) from a sacral bone of a human body to a surface of a toe is divided by a transmission velocity (v) of a nerve which passed through the sacral bone of the human body or the vicinity of the sacral bone.

2. The electrical stimulation therapeutic device according to Claim 1 which includes
a switch portion in which a connection of the detection electrode with the myoelectric signal processing portion is turned on or off, and
a myoelectric signal selecting portion which controls the switch portion so that, during the detection stop period of time, the connection of the detection electrode with the myoelectric signal processing portion can be turned off and also, at the end of the detection stop period of time, the connection of the detection electrode with the myoelectric signal processing portion can be turned on.

3. The electrical stimulation therapeutic device according to Claim 2, wherein
the myoelectric signal selecting portion makes a potential of the switch portion into a reference potential or a pre-held drift potential or makes the switch portion into an open state during the detection stop period of time.

4. The electrical stimulation therapeutic device according to any one of Claim 1 to Claim 3 which includes an integrator by which, before processing of the myoelectric signal in the myoelectric signal processing portion, myoelectric signals detected by the detection electrode are integrated for a predetermined sampling time and the integrated signal is output in the myoelectric signal processing portion as a first signal.

5. The electrical stimulation therapeutic device according to Claim 4, wherein
the myoelectric signal processing portion determines an amplitude of the first signal based on a difference between an averaged value of a first section of the first signal for a predetermined first period of time and an averaged value of a second section of the first signal for a predetermined second period of time temporally before and after the first section.

6. The electrical stimulation therapeutic device according to Claim 5, wherein
the first section and the second section of the first signal are averaged by being subjected to addition arithmetic average processing or moving average processing.

7. The electrical stimulation therapeutic device according to Claim 5 or Claim 6 which includes a stimulation signal output portion that supplies the stimulation signal to the pair of application electrodes and also demarcates an amplitude of the stimulation signal in a stepwise manner, thereby indicating a level of the amplitude of the stimulation signal on the display portion,
wherein
the myoelectric signal processing portion demarcates the first section of the first signal equal to or higher than a predetermined amplitude in a stepwise manner to indicate a level of the amplitude of the first section on the display portion.

8. The electrical stimulation therapeutic device according to any one of Claim 1 to Claim 7 which includes a urination disorder treatment device.

9. An electrical stimulation therapeutic method which uses an electrical stimulation therapeutic device including
a pair of application electrodes which are disposed at the back of a sacral bone of a person to be treated and supplies an electrical stimulation signal from the back of the sacral bone,
a detection electrode which is disposed on a surface of a toe of the person to be treated and detects a myoelectric signal of the toe,
a display portion which determines whether the myoelectric signal of the toe is generated in response to the stimulation signal, and
a myoelectric signal processing portion which processes the myoelectric signal detected by the detection electrode and displays it visually on the display portion,
wherein
the myoelectric signal processing portion does not detect the myoelectric signal of the toe during a predetermined detection stop period of time from output of the stimulation signal, and
the detection stop period of time is set on the basis of a quotient (x/v) obtained when a distance (x) from a sacral bone of a human body to a surface of a toe is divided by a transmission velocity (v) of a nerve which passes through the sacral bone of the human body or the vicinity of the sacral bone,
the electrical stimulation therapeutic method comprising:
a step in which the pair of application electrodes are disposed at the back of a sacral bone of a person to be treated;
a step in which the detection electrode is disposed on a surface of a toe of the person to be treated;
a step in which the electrical stimulation signal is supplied from the pair of application electrodes to the back of the sacral bone; and
a step in which, during supply of the stimulation signal from the pair of application electrodes, whether the myoelectric signal of the toe is generated in response to the stimulation signal is determined by visually confirming the display portion.

10. The electrical stimulation therapeutic method according to Claim 9 which includes a step in which, where a determination is made by visually confirming the display portion that the myoelectric signal of the toe is not generated in response to the stimulation signal, supply of the stimulation signal from the pair of application electrodes is stopped.
